# EUROPEAN PATENT APPLICATION

(11) **EP 1 334 740 A1**
(43) Date of publication of application: **13.08.2003**
(21) Application number: 02425069.8
(22) Date of filing: 11.02.2002
(51) Int. Cl.: A61M 5/32

(54) **Glass safety syringe and relative safety kit for glass syringe**

(71) Applicant: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo

(57) **Abstract**

A safety syringe (100) comprises a glass syringe body (1), a plunger (2) slidable inside the syringe body (1) and provided at the rear with a manually operable stem (20), an injection needle (11) mounted at the front end (10) of the syringe body (1), a sheath (3) mounted integrally on the syringe body (1), a sleeve (4) mounted slidably on the shcath to pass from a retracted use position to an advanced safety position, in which the needle (11) is protected by the sleeve (4), locking means mutually provided in the sheath and in the sleeve to lock the sleeve (4) in position when it is in the retracted use position and in the forward safety position, and operating means (22) integrally mounted with the stem (20) of the piston to cooperate with locking means (43) so as to allow movement of the sleeve (4) from the retracted use position to the advanced safety position.

## Description

The present invention refers to a glass syringe, of the disposable type, provided with an automatic safety device to protect the needle once the injection has been performed. The present invention also refers to a relative safety kit that can be applied to a disposable glass syringe.

A syringe generally comprises a cylindrical body open at the rear to receive a plunger. An internally hollow needle is mounted at the head of the syringe body.

Because of health regulations and to avoid transmission of infectious diseases, syringes must generally be used only once and then discarded. For this reason, there is a growing demand for disposable syringes adapted to prevent further use.

Moreover, syringes generally present drawbacks from the point of view of safety. In fact, once the syringe has been used, the needle remains exposed at the head of the syringe body, with the risk of injuries or accidental needle sticks.

To overcome these drawbacks, plastic disposable syringes having safety devices to cover the needle are available on the market.

Patent application PCT WO 99/37345 describes a disposable safety syringe which has a needle covering sleeve mounted axially on the syringe body and slidable from a retracted position in which it leaves the needle exposed to allow injection, to an extended position in which it completely covers the needle, preventing re-use of the syringe and acting as a protection against accidental needle sticks.

Once the injection has been performed, the sleeve is automatically brought into the extracted safety position, by means of an automatic mechanism and without any intervention by the user.

Glass syringes, on the other hand, are generally designed to be able to be reused. In fact, the glass syringe body can be sterilized at high temperature. For this reason, glass syringes generally do not provide any safety device for protection of the needle once the injection has been performed.

As is known, prefilled syringes in which the solution is already inside the barrel of the syringe, ready for injection, are widely available on the market. However, there exist some solutions such as vaccines, antidotes and the like which cannot be stored in plastic containers. Consequently, said solutions must necessarily be placed in prefilled glass syringes.

For this reason and for reasons of hygiene and practicality, glass syringes of the disposable type exist on the market. Disposable glass syringes have the drawback of not having a safety device for protection of the needle.

An object of the present invention is to eliminate the prior art drawbacks, providing a glass safety syringe that is practical, versatile, economical and simple to make.

Another object of the present invention is to provide such a glass safety syringe of the disposable type that is able to prevent further attempts at use.

Another object of the present invention is to provide such a glass syringe that is extremely safe and able to prevent accidental injury and tampering after use.

These objects are achieved in accordance with the invention with the characteristics listed in appended independent claim 1.

Advantageous embodiments of the invention are apparent from the dependent claims.

The syringe according to the invention comprises a glass syringe body hollow on the inside and open at the front and the rear, a plunger slidable inside the syringe body and provided at the rear with a manually operable stem and an injection needle mounted at the fore end of the syringe body.

To make this syringe a safety syringe according to the invention, a sheath integrally mounted on the glass syringe body and a sleeve slidably mounted on the sheath to pass from a retracted use position in which the needle protrudes forward from the sleeve to be able to perform the injection to an advanced safety position in which the needle is protected by the sleeve.

Locking means and stops are provided reciprocally in the sheath and sleeve such as to lock sleeve in position when it is in the retracted use position and in the advanced safety position. Operating means able to cooperate with the locking means when the plunger is at the end of the injection stroke are fitted to the rear part of the piston stem, to allow the sleeve to be moved from the retracted use position to the advanced safety position.

The advantages of the syringe according to the invention are obvious in that it allows a glass syringe to be made into a safety syringe, by providing the possibility of covering the needle with the sleeve once the injection has been performed.

Further characteristics of the invention will be made clearer by the detailed description that follows, referring to a purely exemplary and therefore unrestrictive embodiment thereof, illustrated in the appended drawings, in which:
Figure 1 is a top plan view of the safety syringe according to the invention, in the position ready for use, with a needle-covering cap mounted at the head of the syringe body;
Figure 2 is an axial sectional view of the safety syringe of Figure 1, in which the syringe body and the piston stem are shown in side view;
Figure 3 is an axial sectional view taken along the sectional plane III-III of Figure 1, in which the piston stem is shown in side view and in the end of injection position and the needle-covering cap has been removed;
Figure 4 is an axial sectional view, like Figure 3, in which the needle is in the safety position;
Figure 5 is a perspective view, illustrating a sheath for covering a syringe body.

The safety syringe according to the invention, denoted as a whole by reference numeral 100, is described with the aid of the figures.

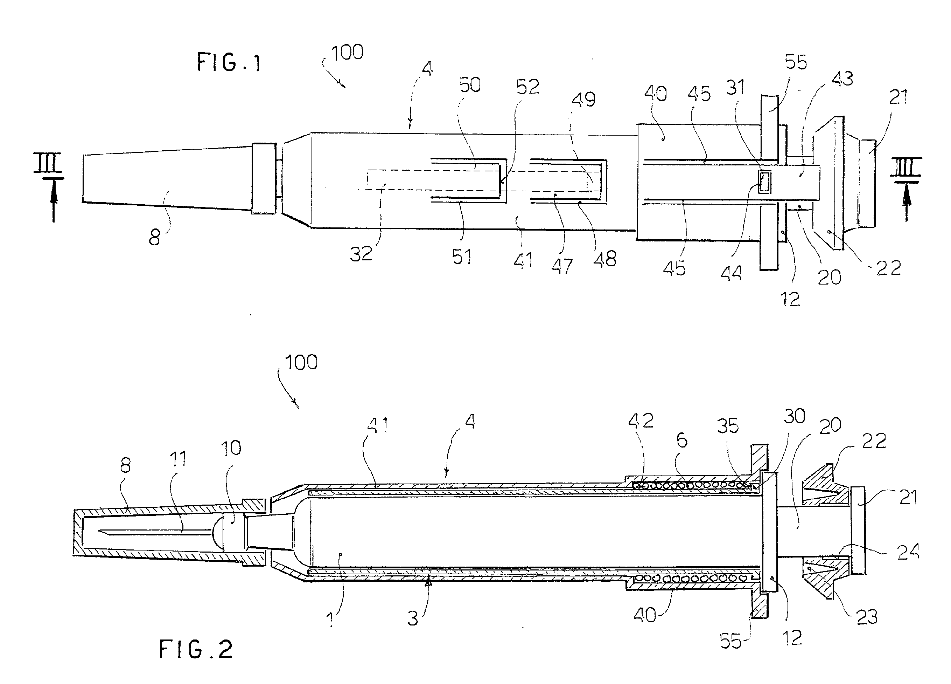

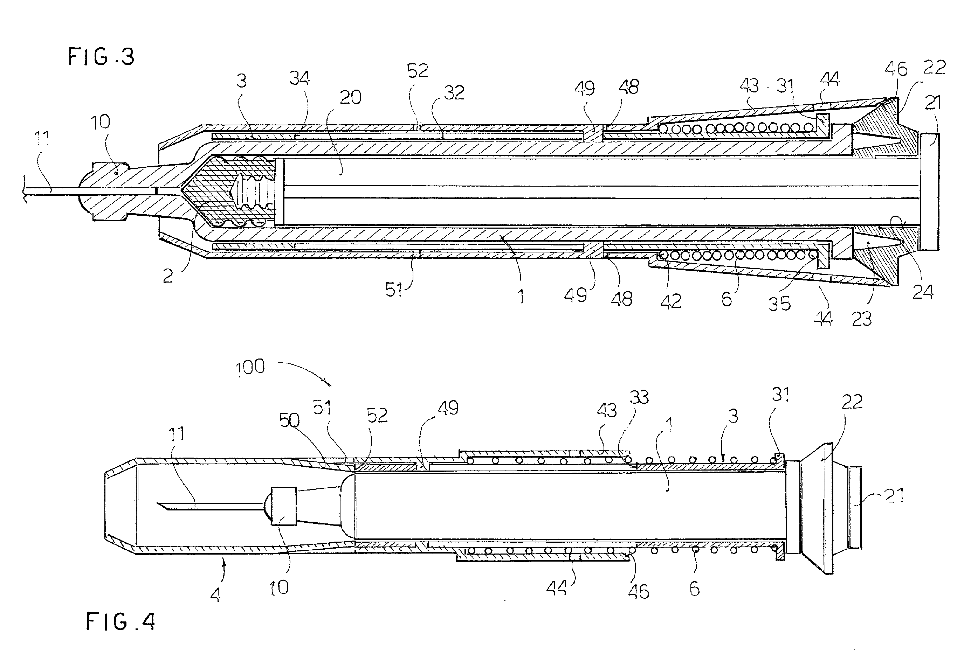

The syringe 100 comprises a syringe body 1 (shown in Figures 2 and 4) made of glass. The syringe body 1 is substantially cylindrical in shape, is hollow on the inside and has a cylindrical chamber open at the front and rear. The body 1 has at its fore end a head 10 into which a needle 11 is sunk. The body 1 has at its rear end a flange 12 that protrudes radially therefrom to facilitate gripping by the user.

As shown in Figure 3, a plunger 2 made of rubber can slide axially with a tight seal in the cylindrical chamber of the syringe body 1. The plunger 2 is supported at the fore end by a stem 20 made of glass or plastic. The stem 20 has at its rear end a flange 21 disposed on the outside of the syringe body 1 to be able to be operated manually by the user.

In the rear part of the stem 20 a frusto conical block 22, disposed around the stem 20, near the flange 21 of the stem, is provided. The frusto conical block 22 has a tapered side surface with a diameter that increases toward the rear part of the stem 20.

The frusto conical block 22 can be formed integrally in the rear part of the stem, or it can be a separate element to apply to the stem 20. In this latter case, the frusto conical block 22 has an axial hole 24 to be forcibly inserted, from the front part of the stem, until it abuts against the rear flange 21 of the stem. In this case, the frusto conical block 22 has an annular groove 23 to be able to yield elastically.

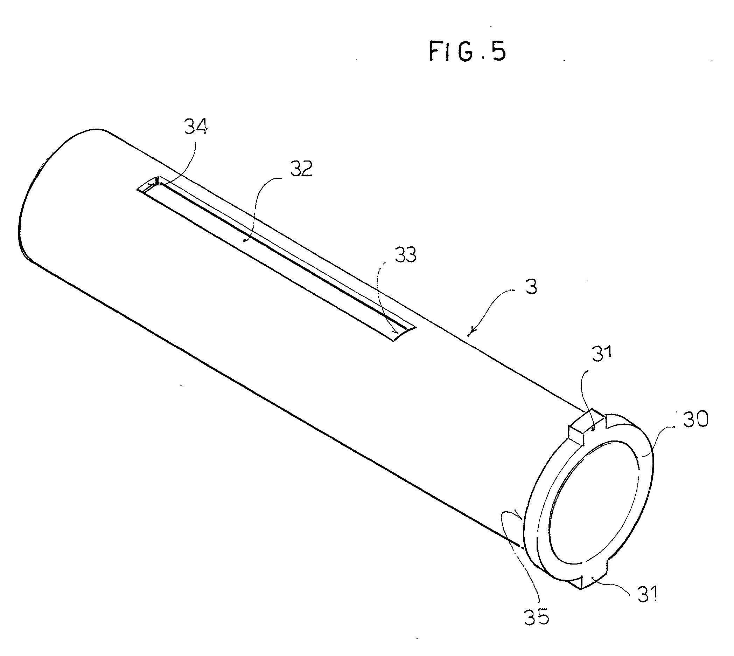

A tubular sheath 3 that almost entirely covers the side surface of the syringe body 1 is mounted on the syringe body 1. As shown also in Figure 5, the sheath 3 comprises at its fore end a collar 30 which protrudes radially outward so as to define an annular abutment surface 35.

Two teeth 31 that protrude radially outward in diametrically opposite directions are provided on the collar 30. Two longitudinal slots 32 disposed in diametrically opposite positions are provided on the side surface of the sheath 3, level with the teeth 31. In this manner, each longitudinal slot 32 defines a rear abutment surface 33 and a front abutment surface 34.

The sheath 3 is made of plastic material and is elastically forced and fixed on the glass body 1. The syringe body 1, with the sheath 3 integral therewith, is mounted axially slidably inside a substantially cylindrical sleeve 4, hollow on the inside. In this manner, the head 10 of the syringe body 1 protrudes forwardly and axially from the sleeve 4 and a needle-covering cap 8 is fitted to the head 10.

The sleeve 4 has a cylindrical rear part 40 with a greater diameter and a cylindrical front part 41 with a smaller diameter, so as to define on the inside an annular abutment surface 42. In this manner, as shown in Figure 2, a helicoidal compression spring 6 which winds around the sheath 3 is interposed between the annular abutment surface 35 of the collar 30 of the sheath 3 and the annular abutment surface 42 of the sleeve 4.

As better shown in Figure 1, two rear tongues 43 are formed in the side wall of the part 40 of the sheath with the largest diameter. The rear tongues 43 are disposed longitudinally in diametrically opposite positions and are flexible. Each rear tongue 43 has a rectangular slot 44 within which the respective tooth 31 of the collar 30 of the sheath snap engages, so as to firmly retain the sheath 3 inside the sleeve 4.

Each rear tongue 43 is defined by two longitudinal parallel notches 45 which reach the end of the part with the greatest diameter 40. In this manner, each rear tongue 43 can bend radially outward. Furthermore, each rear tongue 43 has a rear part which protrudes rearward beyond the rear end of the part with the largest diameter, 40. The rear end of each tongue 43 has a tapered surface 46 able to cooperate with the tapered surface of the frusto conical block 22 of the stem.

At the rear end of the part with the greater diameter 40, a flange 55 protrudes radially to facilitate gripping by the user. The rear flange 55 of the sleeve is not present in the area in which there are the rear tongues 43 so as not to interfere therewith. The flange 55 of the sleeve has in its rear wall an annular abutment surface against which the rear flange 12 of the glass body 1 abuts.

In the lateral part of the part with the smallest diameter 41 of the sleeve two central tongues 47 are formed. The central tongues 47 are disposed longitudinally in diametrically opposite positions and are flexible. Each central tongue 47 is defined by a substantially U-shaped notch 48, so as to be able to bend radially outward.

Each central tongue 47 has an inwardly protruding tooth 49 able to engage in the respective longitudinal slot 32 of the sheath 3. In this manner, when the syringe body 1 with the sheath 3 are inserted inside the sleeve 4, the central tongues 47 are widened and then elastically snap back when the teeth 49 engage in the respective slots 32 in the sheath.

Two front tongues 50 are formed in the side wall of the sleeve part 41 with the smallest diameter. The front tongues 50 are longitudinally disposed in diametrically opposite positions. Each front tongue 50 is defined by a substantially U-shaped notch 51, so as to be able to bend radially outward. Under normal conditions, each front tongue 50 converges inwardly and has at its front end an abutment surface 52.

Operation of the syringe 100 according to the invention will now be described.

With reference to Figures 1 and 2, in an initial condition the spring 6 is compressed between the annular abutment surface 35 of the collar 30 of the sheath 3 and the annular abutment surface 42 of the sleeve 4. The teeth 32 of the sheath are engaged in the slots 44 of the rear tongues 43 of the sleeve avoiding any axial movement of the sheath 2 and body 1 assembly with respect to the sleeve 4. Furthermore, the teeth 49 of the central tongues 47 are in abutment against the front abutment surface 33 of the longitudinal slot 32 of the sheath, avoiding any axial rearward movement of the sheath 3 with respect to the sleeve 4.

The chamber of the body 1 is pre-filled with a solution to be injected. Thus, when the injection is performed and the plunger 2 reaches the end of its stroke in the chamber of the syringe body, as shown in Figure 3, the tapered surface of the frusto conical block 22 cooperates with the tapered surface of the rear end 46 of the rear tongues 43, causing outward radial bending thereof.

Consequently, the teeth 31 of the sheath 3 disengage from the slots 44 of the tongues 43 and the sleeve 4 is no longer firmly constrained to the sheath 3 and syringe body 1 assembly. As a result, as shown in Figure 4, the spring 6 which was compressed is released, lengthening and causing axial forward sliding of the sleeve 4 with respect to the sheath and syringe body assembly, until the teeth 49 of the central tongues 47 of the sleeve abut against the respective rear abutment surfaces 34 of the longitudinal slots 32 of the sheath.

Said axial movement of the sleeve 4 with respect to the sheath and syringe body assembly is guided, since the teeth 49 of the central tongues 47 of the sleeve slide in the respective longitudinal slots 32 of the sheath. Furthermore, when the rear end 52 of the front tongues 50 of the sleeve passes the front end of the sheath 3, the front tongues 50 converge elastically inward.

In this situation, the needle 11 is protected in the safety position inside the sleeve 4. It should be noted that when the syringe 100 is in the safety position, any axial movement of the sleeve 4 with respect to the sheath and syringe body assembly is prevented. In fact, the front end of the sheath 3 abuts against the rear end 52 of the front tongues 50 which are convergent and the rear abutment surfaces 34 of the slots 32 in the sheath are in abutment against the respective teeth 49 of the central tongues 47 of the sleeve.

It is evident that the present invention, besides referring to the safety syringe 100 as described, also refers to a safety kit comprising the sheath 3, the spring 6, the sleeve 4 and the frusto conical block 22, considered as separate pieces which are applied to a glass syringe of the known type to make it a safety syringe.

Numerous variations and changes of detail within the reach of a person skilled in the art can be made to the present embodiment of the invention, without thereby departing from the scope of the invention as set forth in the appended claims.

## Claims

1. A safety syringe (100) comprising:
- a glass syringe body (1) hollow on the inside and open at the front and rear,
- a plunger (2) slidable inside the syringe body (1), said plunger being provided at the rear with a manually operable stem (20) brought out of the syringe body by means of the rear end (21) thereof, and
- an injection needle (11) mounted at the front end (10) of the syringe body (1),
**characterized in that** it comprises:
- a sheath (3) integrally mounted on said glass syringe body (1),
- a sleeve (4) slidably mounted on said sheath to pass from a retracted use position in which the needle (11) protrudes forward from said sleeve to be able to perform the injection to an advanced safety position in which the needle (11) is protected by said sleeve (4),
- locking and stop means, mutually provided in said sheath and in said sleeve to lock said sleeve (4) in position when it is in said retracted use position and said advanced safety position, and
- operating means (22) integrally mounted to the rear part of the plunger stem (20) to cooperate with said locking means, when the plunger (2) is at the end of the injection stroke to allow movement of said sleeve (4) from the retracted use position to the advanced safety position.

2. A safety syringe (100) according to claim 1, **characterized in that** it comprises spring means (6) interposed between said sleeve (4) and said sheath (3).

3. A safety syringe (100) according to claim 2, **characterized in that** one end of said spring means is in abutment against an annular abutment surface (42) defined between a larger diameter part (40) and a smaller diameter part (41) of said sleeve and the other end of said spring means is in abutment against an annular abutment surface (35) defined by a collar (30) protruding outward at the fore end of said sheath (3).

4. A safety syringe (100) according to any one of the preceding claims, **characterized in that** said locking means comprise two teeth (31) radially protruding from said sheath, in diametrically opposite directions, to engage in respective slots (44) provided in two rear elastic tongues (43) longitudinally formed in the rear part of the side wall of said sleeve (4).

5. A safety syringe (100) according to claim 4, **characterized in that** each of said rear tongues (43) is defined by two longitudinal parallel notches formed in said side wall of the sleeve, up to the rear end of the sleeve, and the rear end of said tongues (43) protrudes radially beyond the rear end of the sleeve.

6. A safety syringe (100) according to claim 4 or 5, **characterized in that** said operating means comprise a frusto conical block (22) cooperating with said rear tongues (43), when the plunger (2) reaches the end of the injection stroke, in order to make them diverge outward to disengage said teeth (31) of the sheath from said slots (44) of the rear tongues.

7. A safety syringe (100) according to any one of the preceding claims, **characterized in that** said stop means comprise a pair of flexible central tongues (47), longitudinally provided in diametrically opposite positions in the central part of the side wall of said sleeve (4), said central tongues (4) being provided with respective teeth (49) protruding inward to engage in respective longitudinal grooves (32) provided in said sheath (3).

8. A safety syringe according to claim 7, **characterized in that** each of said longitudinal grooves (32) in the sheath (3) has a rear abutment surface (33) which abuts against the respective tooth (49) of the central tongues of the sheath when said sheath (4) is in the retracted use position and a front abutment surface (34) that abuts against the respective tooth (49) when said sleeve (4) is in the advanced safety position.

9. A safety syringe (100) according to any one of the preceding claims, **characterized in that** said stop means comprise a pair of flexible rear tongues (50), longitudinally provided in diametrically opposite positions in a rear part of the side wall of said sleeve, said central tongues (50) being inwardly convergent and having an abutment surface (52) able to abut against the front end of said sheath (3), when the sleeve (4) is in said advanced safety position.

10. A safety syringe (100) according to claims 7 and 9, **characterized in that** each of said central tongues (47) and each of said front tongues (50) is defined by a respective substantially U-shaped notch (48, 51) formed respectively in the central part and in the front part of the side wall of said sleeve.
